(19) Europäisches Patentamt
European Patent Office
Office européen des brevets

(11) **EP 1 242 102 B1**

(12) **EUROPEAN PATENT SPECIFICATION**

(45) Date of publication and mention
of the grant of the patent:
**24.05.2006 Bulletin 2006/21**

(21) Application number: **00909548.0**

(22) Date of filing: **02.03.2000**

(51) Int Cl.:
*A61K 36/00* (2006.01)   *A61K 35/74* (2006.01)
*A61P 11/06* (2006.01)   *A61P 17/00* (2006.01)
*A61P 31/16* (2006.01)   *A61P 35/00* (2006.01)

(86) International application number:
**PCT/IB2000/000222**

(87) International publication number:
**WO 2000/051580 (08.09.2000 Gazette 2000/36)**

(54) **ASTHMA/ALLERGY THERAPY THAT TARGETS T-LYMPHOCYTES AND/OR EOSINOPHILS**

ASTHMA- UND ALLERGIEBEHANDLUNG DIE AUF T-LYMPHOZYTEN UND/ODER EOSINOPHILE AUSGERICHTET IST

TRAITEMENT DE L'ASTHME/ALLERGIE CIBLANT LES LYMPHOCYTES T ET/OU LES EOSINOPHILES

(84) Designated Contracting States:
**AT BE CH CY DE DK ES FI FR GB GR IE IT LI LU MC NL PT SE**

(30) Priority: **02.03.1999 GB 9904777**
**08.06.1999 GB 9913341**

(43) Date of publication of application:
**25.09.2002 Bulletin 2002/39**

(73) Proprietors:
• **Al-Jassim, Rawaa**
**Napperville,**
**Illinois 60565 (US)**
• **Al-Kaisi, Ban**
**Miami, FL 33176 (US)**
• **James, David**
**Newark,**
**Notts NG24 3EG (GB)**
• **Nedaa Abdul-Ghani Nasif**
**College Road**
**Harrow**
**Middlesex HA1 1GN (GB)**
• **Ahmad, Nagham Zuhair**
**72764 Reutlingen (DE)**
• **Fadhel, Ali Zuhair A.**
**Kalamazoo, MI 49009 (US)**
• **Ahmad, Zeena Z.**
**4881 DC  Zundert (NL)**
• **Ahmad, Noor Z.**
**Old Airport Area, Doha (QA)**

(72) Inventor: **Nasif, Nedaa Abdul-Ghani**
**Harrow, Middlesex, HA1 1GN (GB)**

(74) Representative: **Cronin, Brian Harold John**
**Cronin Intellectual Property**
**Route de Clémenty 62**
**1260 Nyon (CH)**

(56) References cited:
• **ROJO, J. M. ET AL: "Enhancement of lymphocyte proliferation, interleukin-2 production and NK activity by inmunoferon (AM-3), a fungal immunomodulator: variations in normal and immunosuppressed mice" INT. J. IMMUNOPHARMACOL. (1986), 8(6), 593-7, XP000964668**
• **ELKADI A; KANDIL O; TABUNI A-M: "NIGELLA-SATIVA AND CELL-MEDIATED IMMUNITY" ARCHIVES OF AIDS RESEARCH, vol. 1, 1987, pages 232-233, XP000964740**
• **CANAVETE M L ET AL: "EFECTO DE UN NUEVO INMUNOMODULADOR SOBRE LA FUNCIONALIDAD DE MACROFAGOS DE RATON" REVISTA CLINICA ESPANOLA, MADRID,ES, vol. 173, no. 3, 1984, pages 159-162, XP000964667 ISSN: 0014-2565**

- DATABASE BIOSIS [Online] BIOSCIENCES INFORMATION SERVICE, PHILADELPHIA, PA, US1992 VILLARRUBIA V G ET AL: "THERAPEUTIC RESPONSE ON CHRONIC ACTIVE HEPATITIS B CAHB TO A NEW IMMUNOMODULATOR AM3 IMMUNOHEMATOLOGICAL EFFECTS" Database accession no. PREV199293138017 XP002154933 & IMMUNOPHARMACOLOGY AND IMMUNOTOXICOLOGY, vol. 14, no. 1-2, 1992, pages 141-164, ISSN: 0892-3973
- SIDWELL R W ET AL: "ANTIVIRAL AND IMMUNOMODULATING INHIBITORS OF EXPERIMENTALLY- INDUCED PUNTO TORO VIRUS INFECTIONS" ANTIVIRAL RESEARCH,NL, ELSEVIER SCIENCE BV., AMSTERDAM, vol. 25, no. 2, 1994, pages 105-122, XP000600991 ISSN: 0166-3542
- DATABASE BIOSIS [Online] BIOSCIENCES INFORMATION SERVICE, PHILADELPHIA, PA, US1997 VILLARRUBIA V G ET AL: "The immunosenescent phenotype in mice and humans can be defined by alterations in the natural immunity reversal by immunomodulation with oral AM3." Database accession no. PREV199799456612 XP002154934 & IMMUNOPHARMACOLOGY AND IMMUNOTOXICOLOGY, vol. 19, no. 1, 1997, pages 53-74, ISSN: 0892-3973
- DATABASE BIOSIS [Online] BIOSCIENCES INFORMATION SERVICE, PHILADELPHIA, PA, US1990 SCAGLIONE S ET AL: "IMMUNOFERON AM3 IN TREATMENT AND PREVENTION OF RESPIRATORY INFECTIONS IN AGED PATIENTS AFFECTED WITH CHRONIC BRONCHOPNEUMOPATHY" Database accession no. PREV199038094542 XP002154935 & GERIATRIKA (MADRID), vol. 6, no. 1, 1990, pages 64-69, ISSN: 0212-9744
- SANCHEZ PALACIOS A ET AL: "[Immunologic clinical evaluation of a biological response modifier, AM3, in the treatment of childhood infectious respiratory pathology]. Valoracion clinica inmunologica de un modificador de la respuesta biologica, AM3, en el tratamiento de la patologia respiratoria infecciosa infantil." ALLERGOLOGIA ET IMMUNOPATHOLOGIA, (1992 JAN-FEB) 20 (1) 35-9., XP000971177
- VILLARRUBIA V.G. ET AL: "Therapeutic impact of AM3 (biological response modifier) on prevention of recurrent acute infections of the respiratory tract. An analysis of 683 persons at high risk." ACTA TOXICOLOGICA ET THERAPEUTICA, (1993) 14/3 (121-134)., XP000971156

**Description**

TECHNICAL FIELD.

This invention is generally directed to the fields of medicine and pharmacology, and specifically directed to a pharmaceutical composition for the treatment of asthma/allergy, consisting essentially of glycophosphopeptical which is active to stimulate T-helper lymphocytes type 1 therefore selectively switching-off the eosinophil inflammation.

REFERENCES

Are listed according to their appearance in the text, at the end of the description.

BACKGROUND ART

Asthma is the epidemic of the new millennium. Despite the increase in our knowledge, the morbidity, mortality and prevalence of asthma and other allergic diseases are increasing as shown by WHO statistics. (1)

Barnes J December 1999, reviews the current state of anti-asthma therapy. Over the past 10 years there have been striking improvements in the treatment of asthma largely as a result of the earlier and more widespread use of inhaled corticosteroids. The development of new treatments for asthma has proved difficult, although several immunologic approaches are undergoing preclinical and clinical assessment. Antileukotrienes are the only new class of drugs to treat asthma that have been introduced in the past 25 years, but their efficacy is somewhat limited and unpredictable, as compared with that of inhaled corticosteroids. (2)

The main disadvantage associated with inhaled corticosteroids is that they should be used on day-to-day bases, as alternate day steroid therapy is unable to control the disease (British National Formulary). Four weeks treatment with recommended dose of corticosteroids is associated with significant increase in peak expiratory flow rate, and decreases the need for rescue salbutamol use in asthmatic subjects, but was not associated with large reductions in markers of eosinophilic inflammation, bronchovascular permeability, or mucus hypersecretion. Alternative therapies for corticoseroid-dependant asthma, such as methotrexate, cyclosporine and oral gold, are problematic and have high incidence of adverse effect. (2)

2-Immunological therapy: in the form of allergen immunotherapy is the only therapeutic mode which has the potential to modify the natural course of the disease, hence, immunotherapy is a preventive and curative treatment, mostly by inducing immune deviation (upregulation of a distinct subset of Th0/Th1 cells). It is a cumbersome therapy.

There is accordingly an outstanding need for an effective and convenient means for treating and/or preventing type I IgE-mediated hypersensitivity reactions, including asthma, in mammals.

Glycophosphopeptical: The present inventor has, surprisingly, found that a short-term administration of glycophosphopeptical (glicofosfopeptical) to patients suffering from asthma is capable of treating and/or preventing asthma. Glycophosphopeptical is marketed under the trade names "IMMUNOFERON" and "INMUNOFERON" drug by Industrial Farmaceutica Cantabria, S. A. (Spain). Glycophosphopeptical is a GLUCOMANNAN from Candida utillis to be used as an immuno-stimulant for oncology, secondary immunodeficiency, and stimulating cell mediated immunity. It is not indicated for the treatment of diseases caused by type I hypersensitivity and asthma defined.

Of particular interest was the clinical effect of glycophosphopeptical treatment in chronic bronchitis patients, which resulted in significant increases in the number of monocytes as well as their phagocytic and chemotactic activity. The depressed number of natural killer cells was reversed to near their levels in young healthy adults. These observations help to explain how glycophosphopeptical aids in the restoration of natural cellular immunity and its possible application as an adjuvant to bacterial & viral vaccines as well as in the treatment chronic bronchitis. (3)

Inmunoferon enhances the activities of early-type interferon inducers and natural killer cells, although it is not an interferon inducer by itself. (4)

Intraduodenal administration of a phosphorylated glycomannan-protein fraction of the cell wall of Candida albicans resulted in a significant increase in Interferon response in the abdominal lymph in rabbits immunized against Candida albicans. Antiviral activity was absent in plasma in all cases. (5)

Glocomannan- I containing antiobesity pharmaceuticals due to delayed food digestion in the stomach. (6)

Pharmaceutical application of Konjac glucomannan is disclosed in reference. (7)

The immunologic enhancement activity of dicarboxy glucomannan was evaluated in vitro by I determining glucose consumption and beta-glucuronidase activity in cultured macrophages. (8)

DISCLOSURE OF THE INVENTION

The invention is based on my novel concept in immunopathology of allergy, as follows :

> A normal person is in a state of "Tolerance to Environmental Antigen, TEA". Pre-inflammatory phase of allergy is controlled by Th1 cells, and it's cytokine interferon. This is based on my discovery that interferon is a potent Eosinophil Chemotactic Factor.
>
> TH1 suppression is the cause of allergy. Manifested by low serum interferon in acute asthmatic attacks. (9, 10)
>
> Th1 stimulation is capable of selectively switch-off the eosinophilic airway inflammation, normalizing serum interferon This can be achieved by using a novel class of asthma therapy, which is the subject of this invention. 5"days" therapy with a BCG-like Th1 stimulation ⟶ long term clinical remission.

The invention therefore provides for the use of glycophosphopeptical in the manufacture of a medicament for the treatment and/or prophylaxis of asthma/allergy in a mammal such as a human.

This use of glycophosphopeptical can be directed to the manufacture of a medicament for the treatment of intrinsic asthma or mixed intrinsic/extrinsic asthma, or to the manufacture of a medicament for the treatment and/or prophylaxis of type I hypersensitivity reaction/allergy. In the latter case the manufacture of the medicament can be for the treatment and/or prophylaxis of extrinsic asthma, or for the treatment and/or prophylaxis of allergic and perrenial rhinitis, allergic conjunctivitis, chronic urticaria, atopic dermatitis and/or laryngeal oedema.

In all cases, the medicament can be presented in a form for short term therapy by administration over a period of 1 to 30 days, preferably over 3 to 5 days, to produce a long term clinical remission of the type I hypersensitivity reaction/allergy over a period of months.

For instance, the medicament is presented as capsules, tablets, lozenges, a powder or a syrup for oral administration, in doses of up to 2000 mg, preferably about 200-1000 mg of the active agent glycophosphopeptical.

Alternatively, the medicament is presented for topical administration in the form of eye or nasal preparations such as drops or ointment, or skin or vaginal preparations such as cream and ointment.

The present invention thus introduces a new class of anti-allergy/anti-asthma therapy that targets the pre-inflammatory phase of the allergic reaction being defined by the present inventor as "Th1 lymphocytes" and its cytokine "interferon".

This present invention provides for the manufacture of a pharmaceutical composition and treatment of asthma/allergy, consisting essentially of Glycophosphopeptical which is active to stimulate T-helper lymphocytes type 1 therefore selectively switching-off the eosinophilic inflammation.

This, surprisingly, leads to a method of treatment for patients suffering from asthma/allergy, by administering Glycophosphopeptical to a mammal such as human in need of such treatment a shot of 5 days only, to get a significant decrease in symptom score started day 3, and in sputum eosinophils by day 14, followed by long-term clinical remission of a mean of 6 months, therefore this method is capable of treating and/or preventing the disease.

The present invention specifically leads to a method of treatment of diseases caused by type I IgE-mediated hypersensitivity reaction comprising the administration to a mammal such as a human in need of such treatment, of an effective dose of glycophosphopeptical as Th1 stimulating agent.

The present invention is specifically typified by a dosage regimen as a characterizing feature, that involves administering to a patient suffering from a chronic disease a short-term therapy of 1-30 days, preferably 5 days, of glycophosphopeptical as Th1 stimulating agent, to get a long-term clinical remission of months as a result of selective switching-off of the eosinophilic inflammation.

The present invention is specifically directed to the use of glycophosphopeptical as Th1 stimulating agent for the preparation of an agent for the treatment and/or prophylaxis of diseases caused by type I IgE-mediated hypersensitivity reaction, such as extrinsic, intrinsic or mixed asthma, allergic and perennial rhinitis, allergic conjunctivitis, chronic urticaria, atopic dermatitis, and/or laryngeal oedema, to be administered to a mammal such as human in need of such treatment.

The present invention is still further directed to the use of glycophosphopeptical as Th1 stimulating agent, for the treatment and/or prophylaxis of diseases caused by type 1 IgE-mediated hypersensitivity reaction for administration to a mammal such as a human.

The present invention is specifically directed to a medicament characterized in that said Th1 stimulating agent comprises Glycophosphopeptical in free base form, or a pharmaceutically acceptable salt or hydrate, or any pharmacologically active form.

The present invention claims a medicament which is adapted and/or packaged for periodic administration to said mammal in doses over a period of 3- 30 days, preferably 5 days in doses at least once daily up to ten times/day. The said medicament is characterized in that each one of said doses comprises up to 2000 mg of said active agent, preferably about 200-1000 mg, of said active agent, adapted for oral administration to said mammal in capsules, or tablets, or lozenges, or as a powder, or a suspension, or a syrup. Also a medicament which is adapted for topical administration to said mammal such as a human, in the form of eye or nasal drops or ointment, also skin or vaginal cream or ointment. Each of the above preparations are to be produced as a kit packaged in separate doses for periodic administration to said mammal such as a human, contains written or printed instructions.

The use of glycophosphopeptical as Th1 stimulating agent in the treatment of allergy/asthma is dependent on the fact that interferon is an in vivo Eosinophilic Chemotactic Factor, and that serum interferon and Th1 lymphocytes are controlling the pre-inflammatory phase of allergic reaction.

The method of treating chronic asthma and allergy using 5 days schedule is based on that the recommended dose of Th1 lymphocytes stimulating agent is sufficient to selectively switch-off the eosinophilic inflammation in the patient's airway.

Th1 lymphocytes stimulating agents, are capable of stimulating T lymphocytes in culture, comparable to Purified Protein Derivative of BCG, as a standard agent that stimulates Cell Mediated Immunity.

The present invention is providing a shot therapy of 1-20 days, preferably 5 days for type I hypersensitive reaction, of particular interest but not limited to the chronic corticoseroid — dependent allergy and asthma. It provides a steroid-saving activity.

The present invention is providing a shot therapy of 1-20 days, preferably 5 days for chronic diseases, the therapeutic effect of which lasts for a mean of 6 months. Such therapeutic schedule had never been known in medical practice.

## INDUSTRIAL APPLICABILITY

Manufacturing a pharmaceutical preparation to provide a therapy for mammals including humans for the treatment of asthma and allergy, containing the active ingredient glycophosphopeptical, that may be administered orally as capsules, tablets, slow release preparations, or liquid preparation, also parenteral preparations and topical medicaments for nasals, eyes, or skin and vaginal preparations.

## BEST MODE FOR CARRYING OUT THE INVENTION.

The present invention was conceived during October 1993, after an experiment of nature that happened to the inventor. Being severe asthmatic her asthma was relieved after a certain health incident. As an immunologist she linked the incident with interferon. This is considered as Stage I.

Stage II: The discovery that interferon is a potent in vivo Eosinophil Chemotactic Factor.

Stage III: A marketed drug inmunoferon (glycophosphopeptical), indicated for diseases unrelated to type 1 hypersensitivity, was linked with allergy in a novel way (depending on the above observation), using it in a non-routine indication and dosage.

1- In order to prove its utility and reduction to practice, a double-blind placebo controlled clinical trial was designed. 120 subjects with different types of allergy were chosen and divided into two groups, matched for age, sex, and severity of the allergic condition after an informed consent into the study. Group 1 included 60 patients treated with inmunoferon. Group 2 included 60 patients treated with placebo.

Diseases involved include seasonal allergic rhinitis, allergic conjunctivitis, chronic urticaria, asthma, and laryngeal edema.

2- The duration of treatment, the total dose received and the schedule of therapy were verified to find the best method of treating various allergies. Glycophosphopeptical was given in addition to the conventional therapy. The full course of 15 g total dose, was divided over 5 days administering 1000 mg 8 hourly.

Alternatively a single dose of 500 mg glycophosphopeptical, a single dose of 1000 mg glycophosphopeptical, or one day therapy. Any of this treatment can be repeated on need.

3- The patients were self-evaluated daily regarding symptoms severity over the preceding 24 hours. A global overall evaluation of treatment efficacy was made by the doctor according to daily notes and findings during clinical examination, at intervals, depending on patient's attendance.

4- The response was recorded according to, the time of onset, and the degree of a noticeable symptomatic improvement.

5- Every one of the patients included was having severe symptoms which are sufficiently troublesome to interfere with daily activity or nocturnal sleep.

The final global improvement rating includes:

- **-** Markedly improved: almost approaching normal condition.
- **-** Moderately improved: having mild symptoms.
- **-** Slightly improved: having frequent troublesome symptoms but not interfering with daily activity or sleep.
- **-** Unchanged: remains as in the pre-treatment condition.
- **-** Difficult to evaluate: no conclusion could be reached.

6- Three main symptoms were chosen for each of the conditions studied, they were:

- **-** In seasonal allergic rhinitis: running nose, frequency of sneezing, nasal obstruction.
- **-** Allergic conjunctivitis: redness of the eye, itching, swelling.
- **-** Chronic urticaria: frequency of recurrence, distribution on the body, severity of itching.
- **-** Asthma: dyspnoea, wheeze, and cough.
- **-** Laryngeal edema: fullness in the throat, hoarseness of the voice, inspiratory diffculty.

During the course of Glycophosphopeptical treatment, 80% of the treated patients showed a significant decrease in symptom score in the treated group compared to placebo. The onset of action is within 8 hours, 50% reduction in symptom score was noticed by day 3, reaching maximum in day 7. Such symptomatic improvement is totally unexpected particularly in patients with allergic rhinitis, asthma and laryngeal edema.
Above all, is the observation that a long-term effect for this short-term therapy was noticeable! During glycophosphopeptical treatment it was possible to stop all other forms of therapy, including steroids. Hence the present invention is useful as a treatment and/or prevention of allergy and asthma.
Side effects: few are mentioned in the manufacturer's leaflet; glycophosphopeptical is not contraindicated for asthma or allergy, no other side effects were noticed during this short course of therapy.
Stage IV: Nine patients age range 36-72 with chronic severe asthma of a duration ranging between I-32 years, all of whom were on a maximal dose of broncodilators (as recommended by the manufacturer) and maintenance corticosteroids, were chosen on account of poor response to conventional treatment, were treated according to the present invention administering glycophosphopeptical orally as in the following design of study:

Day 0: pre-treatment period, is considered as base line, patients were receiving maximal dose of broncodilators (as recommended by the manufacturer) and maintenance corticosteroids.

Day 1: is the beginning of glycophosphopeptical treatment, 1000 mg glycophosphopeptical is administered to the patient 8 hourly, for 5 days (total of 15 grams or 30 capsule) over the whole study period.

The patients were asked to refrain from taking their conventional drugs and steroids when possible.
The effects of treatment with Glycophosphopeptical on the subjects were assessed according to the following assessment criteria:
I-Symptom triad of cardinal symptoms including dyspnoe, cough, and sputum. The symptoms were scored daily as follows:

| | | |
|---|---|---|
| Dyspnoea: | No dyspnoea | Score = 0 |
| | Mild on doing physical activity | Score = 1 |
| | Moderate at rest | Score = 2 |
| | Severe constant annoying dyspnoea | Score = 3 |
| Cough: | No cough | Score = 0 |
| | Mild cough at times | Score = 1 |
| | Moderate frequent annoying cough | Score = 2 |
| | Severe constant distressing cough | Score = 3 |
| Sputum: | No sputum | Score = 0 |
| | Small amount expectorated with ease | Score = 1 |
| | Tenacious moderate amount | Score = 2 |
| | Plenty causing severe mucus-related symptoms | Score = 3 |

2-Composite symptom scoring:
This is an indication of therapeutic effectiveness in improving the patients' global assessment. The sum of the score is 39. Scorings on a level of 0-3 being awarded for each of the following symptoms, the score was recorded daily:
Cough frequency, cough severity, audible wheeze, Tachycardia, Chest discomfort, Nocturnal dyspnoea disturbing sleep, Ability to walk up stairs, Ability to talk and laugh, Stress incontinence caused by the cough, Necessity to take days off work, Psychological well-being, Hospitalization rate, and need for conventional drugs.

The percent reduction in symptom scoring was calculated according to the following formula:

$$\text{(base-line score - follow-up score)/(base-line score} - 1)\%.$$

3-Pulmonary function test:

Was carried out to assess "the alteration in airway flow and bronchial patency resulting from glycophosphopeptical treatment" by measuring changes in FEVI, PEFR, FEF25% (alveolar), FEF50% (small airways), FEF75% (large airways).

4-Sputum examination:

Hypersecretion of heavy mucus or sputum, resulting in mucus-related symptoms, is characteristic of asthma. The eosinophil levels in the sputum are generally found to correlate with the severity of the disease. The sputum produced by the patients during the course of glycophosphopeptical therapy was consequently observed for changes both at a macroscopic and a microscopic level

Macroscopically, there was a tremendous decrease in the amount of sputum and a change in its visco-elastic properties. The sputum became thin and easily expectorated. Changes were noticeable after day 3 of the therapy, and within 10 days the patients were free from mucus-related symptoms.

Serial microscopic sputum examinations for the percentage of eosinophils in relation to other inflammatory cells were performed. Samples of sputum from the patients were smeared onto slides, fixed with methanol, and stained using haematoxylin-eosin. A total of 300 inflammatory cells (lymphocytes, polymorphonuclear leukocytes, macrophages, and basophils) were counted in each slide, and the percentage of eosinophils in this count was calculated.

The percent reduction in sputum eosinophils was calculated according to the following formula:

$$\text{(base-line count - follow-up count)/(base-line count} - 1)\%.$$

5- Serum samples to carry out relevant tests as serum interferon level, total serum IgE, and serum cationic protein.

Results of stages III and IV:

- The total number of asthmatic patients treated with glycophosphopeptical is: 25 patients in stage III + 10 patients in stage IV + 20 patients during the year 1999.
- The total number of controls: 35 patients.

The percent reduction in symptom score was 65-100%

The reduction in symptom score as a result of glycophosphopeptical therapy is shown in table I

Table 1:

| Mean symptom score | Glycophosphopeptical (N=55) | Placebo (N=35) |
|---|---|---|
| Day 0 | 34.5 | 33.2 |
| Day 3 | 20.5 | 27.3 |
| Day 7 | 9.66 | 29.7 |
| Day 14 | 5.8 | 30.6 |
| Day 21 | 4.4 | 31.4 |

Improvement was noticeable by day 3 of the therapy, and reached a maximum by 7-10 days of therapy

Patient compliance is very good, because this treatment is capable of achieving the confidence of the chronically ill patient with some psychological element from the disease.

Tapering of oral and inhaled corticosteroid was possible, this treatment have a steroid saving effect. In addition rescue use of inhaled bronchodilators as measured by the daily number of puffs required was significantly reduced.

The increment in airway patency is the result of the selective switching-off of the eosinophilic inflammatory process. There was a decrease in the percentage of sputum eosinophils with glycophosphopeptical therapy from 80% to less than 10% within the first two weeks of glycophosphopeptical therapy. In addition the use of student t test shows significant decrease in the number of sputum eosinophils after glycophosphopeptical therapy as compared to pre-treatment number.

Long-term follow up:

After the end of the course of glycophosphopeptical therapy, during which a total of 30 capsules of glycophosphopeptical were administered to each subject, no further glycophosphopeptical was administered. Over the next 23 months, the subjects' symptoms were regularly assessed on the following criteria:

Frequency of attacks of shortness of breath, coughing, wheezing, and sputum
Daily activity
Disturbance of sleep
Need for traditional forms of asthma therapy
Rate of hospitalization

Asthmatic attacks were infrequent and mild, being manifested only in some shortness of breath, with mild coughing and small amounts of sputum. Traditional forms of asthma therapy were required only when the subjects were suffering from colds. At least eight out of the ten subjects were able to lead a normal lifestyle during the long-term follow up period, with no nocturnal or early morning dyspnoea. It was found that the hospitalization rate for each subject during the long-term follow up period was on average reduced from several times per month (prior to glycophosphopeptical therapy) to 1-3 times per year.

During the follow-up period, microscopic analysis of the subjects' sputum indicated a reduction in eosinophil levels to only 5-10% of the total inflammatory cells. This level was maintained except during exacerbation, when eosinophil levels rose to 30-40% of total inflammatory cells.

Conclusion: Glycophosphopeptical is an agent that can be used in treating asthma of all types and severity, allergic/ perennial rhinitis, and other allergies. This short-term therapy produce Long-term effect.

<u>REFERENCES</u>

1- Help our children breath. Press release WHO 7.92"/December 1998.World health organization press office, WHO web site: www.who.ch/

2- Barnes P J, The New England Journal of Medicine. Dec 23, 1999. Vol. 341, No. 26:

3- Villarubia , V.G. ; Moreno , Koch M.C. ; Calvo , C. ; Gonzaiez , S. ; Alvarez - Mon, , M. The immunosenescent phenotype in mice and humans can be defined by alterations in the natural immunity reversal by immunomodulation with oral AM3.The immunosenescent phenotype in mice and humans can be defined by alterations in the natural immunity reversal by immunomodulation with oral AM3.. Immunopharmacol Immunotoxicol, 1997;19(1): 53 - 74 .

4- Moya P, Baixeras E, Barasoain I. Inmunoferon enhances the activities of early-type interferon inducers and natural killer cells. Immunopharmacol. Immunotoxicol. 1987, 9 (2-3); 234-56.

5- Paulesu L, Bocci V, Pessina G P, et al. Interferon induction in rabbits after intraduodenal administration of a phosphorylated glycomannan-protein fraction of the cell wall of Candida albicans. Immunol. Lett. 1991, 27 (3); 231-5.

6- Glocomannan -containing antiobesity pharmaceuticals. Shimizu, Hideki (Shimizu Chemical K. K., Japan). Jpn. Kokai Tokkyo Koho JP 01224320 A2 7 Sep 1989 Heisei, 5 PP. (Japan) CODEN: JKXXAF. CLASS: ICM: A61K031-715. ICS: A23K001-16: A61K035-78. APPLICATION: JP 88-48345 1 Mar 1988. DOCUMENT TYPE: Patent CA Section: 63 (Pharmaceuticals) Section cross-reference(s): 1.

7- Jin Miaozhen; Yang, Fan. Pharmaceutical application of Konjac glucomannan. Guangdong Yiyao xueyuan xueyuan xuebae, 10 (1), 1-3 (Chines) 1994.

8- Ohya, Ynichi; Ihara, Kenji; Murata, Jun-ichi; Ouchi, Tatsuro. Bioegradation and immunological enhacement activity of dicarboxy-glucomannan having recognizable branched saccharide residues, Adv. Sci. Technol., 10 (Intelligent Materials and Systems), 273-280 (English) 1995. CODEN:ASETE5. DOCUMENT TYPE:Joumal CA Section: 1 (pharmacology) Section cross-reference(s):44

9- Jankowska-R, Szalaty-H, Malolepszy-J, Sypula-A, Zielinska-Jenczylik-J. Production of interferon by leukocytes from patients with atopic and nonatiopic asthma. Arch-Immunol-Ther-Exp-Warsz. 1988; 36 (5): 523 - 6.

10- WHO Position Paper Allergen immunotherapy: therapeutic vaccines for allergic diseases. Allergy 1998; 53 Supplement 44: 12-13.

**Claims**

1. Use of glycophosphopeptical in the manufacture of a medicament for the treatment and/or prophylaxis of asthma/allergy in a mammal such as a human.

2. The use according to claim 1 of glycophosphopeptical in the manufacture of a medicament for the treatment of intrinsic asthma or mixed intrinsic/extrinsic asthma.

3. The use according to claim 1 of glycophosphopeptical in the manufacture of a medicament for the treatment and/or prophylaxis of type I hypersensitivity reaction/allergy.

4. The use according to claim 3 of glycophosphopeptical in the manufacture of a medicament for the treatment and/or prophylaxis of extrinsic asthma.

5. The use according to claim 3 of glycophosphopeptical in the manufacture of a medicament for the treatment and/or prophylaxis of allergic and perrenial rhinitis, allergic conjunctivitis, chronic urticaria, atopic dermatitis and/or laryngeal oedema.

6. The use claimed in any one of claims 1 to 5, wherein the medicament is presented in a form for short term therapy by administration over a period of 1 to 30 days, preferably over 3 to 5 days, to produce a long term clinical remission of the type I hypersensitivity reaction/allergy over a period of months.

7. The use claimed in claim 6, wherein the medicament is presented as capsules, tablets, lozenges, a powder or a syrup for oral administration, in doses of up to 2000 mg, preferably about 200-1000 mg of the active agent glycophosphopeptical.

8. The use claimed in any one of claims 1 to 5, wherein the medicament is presented for topical administration in the form of eye or nasal preparations such as drops or ointment, or skin or vaginal preparations such as cream and ointment.

**Patentansprüche**

1. Verwendung von Glycophosphopeptical bei der Herstellung eines Medikaments zur Behandlung und/oder Prophylaxe von Asthma/Allergien bei Säugetieren wie z.B. beim Menschen.

2. Die Verwendung gemäß Anspruch 1 von Glycophosphopeptical bei der Herstellung eines Medikaments zur Behandlung von intrinsischem Asthma oder Asthma, das zugleich intrinsisch und extrinsisch ist.

3. Die Verwendung gemäß Anspruch 1 von Glycophosphopeptical bei der Herstellung eines Medikaments zur Behandlung und/oder zur Prophylaxe von hypersensiblen Reaktionen/Allergien vom Typ I.

4. Die Verwendung gemäß Anspruch 3 von Glycophosphopeptical bei der Herstellung eines Medikaments zur Behandlung und/oder Prophylaxe von extrinsischem Asthma.

5. Die Verwendung gemäß Anspruch 3 von Glycophosphopeptical bei der Herstellung eines Medikaments für die Behandlung und/oder Prophylaxe von allergischer und wiederkehrender Rhinitis, allergischer Bindehautentzündung, chronischem Nesselausschlag, atopischer Dermatitis und/oder Kehlkopfödemen.

**6.** Die Verwendung - wie in jedem Anspruch 1-5 beansprucht - des Medikaments in einer Form zur Kurzzeittherapie durch Verabreichung über einen Zeitraum von 1-30 Tage, vorzugsweise über 3-5 Tage, um eine langfristiges Nachlassen klinischer Symptome bei hypersensibler Reaktionen/Allergien vom Typ I über einen Zeitraum von Monaten zu erzielen.

**7.** Die Verwendung gemäß Anspruch 6, bei der das Medikament in Form von Kapseln, Tabletten und Pastillen, Pulver oder Sirup oral in Dosen bis zu 20 000 mg, vorzugsweise ca. 200-1000 mg, des Wirkstoffs Glycophosphopeptical verabreicht wird.

**8.** Die Verwendung gemäß jedem Anspruchl-5, bei denen das Medikament für topische Anwendung in Form von Augen- oder Nasenpräparaten wie Tropfen oder Salbe oder Haut- oder Vaginalpräparaten wie z. B. Creme- oder Salben verabreicht wird.


### Revendications

**1.** Utilisation de glycophosphopeptique pour la production d'un médicament pour le traitement ou la prophylaxie de l'asthme/allergie chez les mammifères comme par exemple l'être humain.

**2.** Utilisation de glycophosphopeptique selon la revendication 1 pour la production d'un médicament pour le traitement ou la prophylaxie d'asthme intrinsèque ou d'asthme mixte intrinsèque/extrinsèque.

**3.** Utilisation de glycophosphopeptique selon la revendication 2 pour la production d'un médicament pour le traitement ou la prophylaxie d'une réaction ou d'une allergie hypersensitivité type 1.

**4.** Utilisation de glycophosphopeptique selon la revendication 3 pour la production d'un médicament pour le traitement ou la prophylaxie l'asthme extrinsèque.

**5.** Utilisation de glycophosphopeptique selon la revendication 3 pour la production d'un médicament pour le traitement ou la prophylaxie de rhinite allergique ou pérenniale, conjonctivite allergique, urticaire chronique, dermatite atopique et/ou oedème laryngé.

**6.** Utilisation selon l'une des revendications 1 à 5, ou le médicament se présente sous une forme destiné à une thérapie à court terme comportant l'administration pendant une période de 30 jours, de préférence pendant 3 à 5 jours, afin d'aboutir à une rémission de la réaction/allergie hypersensitivité de type I sur une période de plusieurs mois.

**7.** Utilisation selon la revendication 6, ou le médicament se présente sous forme de capsules, comprimés, losanges, poudre ou sirop pour administration orale, selon un dosage jusqu'à 2000 mg, de préférence 200-1000 mg de principe actif glycophosphopeptique.

**8.** Utilisation selon l'une des revendications 1 à 5, ou le médicament se présente pour administration topique sous forme de préparations oculaires ou nasales telles que des gouttes ou pommade, ou des préparations dermatologiques ou vaginales, telles que des crèmes et pommades.